# EUROPEAN PATENT SPECIFICATION

(11) **EP 2 705 847 B1**
(45) Date of publication and mention of the grant of the patent: **02.07.2014**
(21) Application number: 12183189.5
(22) Date of filing: 05.09.2012
(51) Int. Cl.: A61K 35/04, A61K 36/00, A61K 9/06, A61K 31/27, A61K 31/4402, A61K 31/573, A61P 17/06

(54) **Composition for treating psoriasis**
Zusammensetzung zur Behandlung von Psoriasis
Composition de traitement du psoriasis

(43) Date of publication of application: 12.03.2014
(73) Proprietor: PSoriasis+Creams Sweden AB, 302 42 Halmstad (SE)
(72) Inventor: Moll Lorca, Ana, 07001 Palma de Mallorca (ES)
(74) Representative: Ström & Gulliksson AB

(56) References cited:
- US-A1- 2007 207 222
- PAGHDAL K V ET AL: "Topical tar: Back to the future", JOURNAL OF THE AMERICAN ACADEMY OF DERMATOLOGY, C.V. MOSBY, ST. LOUIS, MO, US, vol. 61, no. 2, 1 August 2009 (2009-08-01) , pages 294-302, XP026457845, ISSN: 0190-9622 [retrieved on 2009-01-31]
- GOODFIELD M ET AL: "Double-blind, randomised, multicentre, parallel group study comparing a 1% coal tar preparation (Exorex) with a 5% coal tar preparation (Alphosyl) in chronic plaque psoriasis", JOURNAL OF DERMATOLOGICAL TREATMENT, BASINGSTOKE, GB, vol. 15, no. 1, 1 January 2004 (2004-01-01), pages 14-22, XP009147467, ISSN: 0954-6634, DOI: 10.1080/09546630310017843
- H Jimenez Soriano: "Development of a cream from a self-emulsifying base and moisturizing actives a", , 1 July 2001 (2001-07-01), XP055051677, Retrieved from the Internet: URL:http://ac.els-cdn.com/S0014827X0101087 4/1-s2.0-S0014827X01010874-main.pdf?_tid=1 05f9ab0-6ac4-11e2-8fa8-00000aab0f02&acdnat =1359540289_d53150f29846a91889d8e35fc2f025 2a [retrieved on 2013-01-30]
- RICCIATTI-SIBBALD D ET AL: "Dermatologic vehicles", CLINICS IN DERMATOLOGY, J.B. LIPPINCOTT, PHILADELPHIA, PA, US, vol. 7, no. 3, 1 July 1989 (1989-07-01), pages 11-24, XP023113107, ISSN: 0738-081X, DOI: 10.1016/0738-081X(89)90003-5 [retrieved on 1989-07-01]

## Description

### Technical field

The present invention relates to a novel composition for treating psoriasis and a kit comprising such a composition and a second composition having emollient, lubricating and moisturizing effect on the skin. The first composition comprises various active ingredients including a corticosteroid.

### Background

Psoriasis is an autoimmune disease that affects the skin. The name psoriasis has its basis in Ancient Greek, psoriasis roughly meaning "*itching condition*". There are five types of psoriase i.e. plaque, guttate, inverse, pustular, and erythrodermic. Plaque psoriasis (*psoriasis vulgaris*) is the most common one. Plaque psoriasis is commonly seen as red and white hues of scaly patches appearing on the top first layer of the epidermis (skin). Lesions frequently occur on the skin of the elbows and knees. It can however affect any area, including the scalp, palms of hands and soles of feet, and genitals.

The disorder is seen as a chronic recurring condition whose severity varies from minor localized lesions to complete body coverage. Its cause is not fully understood, but it is believed to have a genetic component and local psoriatic changes can be triggered by an injury to the skin known as the Koebner phenomenon. Further, various environmental factors have been suggested as aggravating to psoriasis. Those include stress, withdrawal of systemic corticosteroid, as well as other environmental factors.

While there are many treatments available, psoriasis is a challenge to treat because of its chronic recurrent nature. Further, as the cause of psoriasis is not fully understood, the treatment typically involves alleviating the manifesting symptoms rather than treating the underlying disease. As immunosuppressant medications, such as corticosteroids, can clear, or at least soothe, psoriasis lesions, one of the hypotheses about the process that occurs in the development of the disease is that the disease is an immune-mediated disorder. The excessive reproduction of skin cells is believed to be secondary to factors produced by the immune system.

Despite that the corticosteroid used to treat lesions typically are low-strength corticosteroids, several side effects are usually seen. The corticosteroid causes skin damage, such as skin thinning, changes in pigmentation, easy bruising, stretch marks, redness and dilated surface blood vessels; especially after prolonged use. As psoriasis causes itching, the subject often scratches and thereby damages the sensitive skin. The cuts may further become infected by bacteria, fungi etc.; especially as the corticosteroid is anti-inflammatory, thereby affecting the immune system and delaying its response to microorganisms. Thus, while corticosteroids are effective in treating psoriasis lesions, the treatment still suffers from various drawbacks. Commonly, the psoriasis lesions reoccur shortly after withdrawal of topical corticosteroids.

The treatment of psoriasis also includes the use of bath solutions and moisturizers, mineral oil, and petroleum jelly as they may help to soothe affected skin and reduce the dryness, which accompanies the build-up of skin on psoriatic plaques.

Further medicated creams and ointments, which are applied directly to psoriatic plaques, may be used to treat psoriasis. Such creams and ointments may reduce the underlying inflammation, remove built-up scale, reduce skin turn over, and clear affected skin of plaques. They contain an active component, such as coal tar, dithranol (anthralin), corticosteroids, vitamin D3 analogues (for example, calcipotriol), and retinoids.

As an example, US 2007/207222 discloses a composition including a wax and a therapeutically effective amount of tar for topical treatment of a tar-responsive dermatological disorder, the composition being in liquid or light gel form when at a temperature selected from room temperature and a temperature of skin of a mammal upon application of the composition to the skin of the mammal.

However, there is a need in the art for an effective composition comprising a corticosteroid for treating psoriasis.

### Summary

The present invention seeks to mitigate, alleviate, circumvent or eliminate at least one, such as one or more, of the above-identified deficiencies by providing a dermal composition comprising a tar extract, such as saponified coal tar; a corticosteroid, such as clobetasol propionate (i.e. [17-(2'-chloroacetyl)- 9-fluoro-11-hydroxy-10,13,16-trimethyl-3-oxo-6,7,8,11,12,14,15,16-ctahydrocyclopenta[a]phenanthren-17-yl] propanoate); an antipruritic, such as an antihistamine, e.g. Chlorpheniramine (i.e. 3-(4-chlorophenyl)-N,N-dimethyl-3-pyridin-2-yl-propan-1-amine); an antibiotic, such as an anti-fungal agent, e.g. tolnaftate (i.e. *O-*2-naphthyl methyl(3-methylphenyl)thiocarbamate); urea; an absorption base; water; lanolin; and propylene glycol. Further, the composition may comprise glycerin, a vegetable oil, such as sweet almond oil, ammonium lactate, and/or a preservative.

Another aspect of the invention relates to such a composition for use in treating or soothing psoriasis lesions by applying the composition to areas of the skin affected by psoriasis.

Another aspect of the invention relates to a kit comprising two distinct compositions, the first composition being such a composition as described herein above, and the second composition being composition comprising aqueous hyaluronic acid, an absorption base, and vegetable oil. Further, the second composition may comprise vitamin E, and optionally a fragrance. The vegetable oil may comprise comprises almond oil, argan oil, and/or macadamia oil.

Another aspect of the invention relates to such a kit for use in treating or soothing psoriasis lesions.

Further advantageous features of the invention are defined in the dependent claims. In addition, advantageous features of the invention are elaborated in embodiments disclosed herein.

### Detailed summary of preferred embodiments

The present inventor has devised a dermal composition for treating or soothing psoriasis lesions comprising a corticosteroid and further active ingredients including a tar extract, an antipruritic, an antibiotic and urea. It was found that such a composition was very effective in treating or soothing psoriasis lesions, as the ones seen in subjects suffering from *psoriasis vulgaris.* Further, it was found that the subjects did experience less severe side effects than experienced previously with conventional compositions comprising a corticosteroid as the active ingredient. Without being bound to any theory is believed that active ingredients act in a synergistic manner. Especially, it seems that side effects typically seen with corticosteroid (e.g. skin damage, such as skin thinning, changes in pigmentation, easy bruising, stretch marks, redness and dilated surface blood vessels) are alleviated even if a potent corticosteroid is used. The time period for symptom relief was significantly shorter than the one experienced with a conventional composition comprising a corticosteroid. Further, the subjects remained asymptomatic for a prolonged period of time.

An embodiment thus relates to a dermal composition comprising a tar extract, a corticosteroid, an antipruritic, an antibiotic and urea. Further, the dermal composition comprises water, lanolin, and propylene glycol. In order to obtain a dermal composition to be applied to the skin, the composition also comprises an absorption base such that a stable emulsion may be obtained.

The corticosteroid is preferably a potent corticosteroid, such as clobetasol propionate, betamethasone dipropionate, halobetasol propionate, fluocinonide, diflorasone diacetate, mometasone furoate, halcinonide, or desoximetasone. According to an embodiment the corticosteroid is clobetasol propionate. While less potent corticosteroids typically are used in treating psoriasis topically, it was found that the skin tolerated potent corticosteroids well, when administered in the present composition. Further, it was found that potent corticosteroids were effective in treating or soothing psoriasis lesions.

Further, the dermal composition comprises a tar extract. Tar, or tar extract, is obtained as a by-product by dry distillation of organic materials, such as coal or wood in the absence of oxygen. There are different types of tar extracts including coal tar, wood tar and shale tar. In the present composition, the tar extract is typically a coal tar extract, also known as liquor carbonis detergens. Especially, the coal tar extract may be saponified coal tar. The tar extract provides the composition with keratolytic and antiseptic properties, whereby counteracting the skin damaging effect of the corticosteroid. Further, the antiseptic effect of the tar extract may prevent the sensitive skin from becoming infected.

The dermal composition also comprises urea. Urea does not influence the emulsion's stability, but it is important for its therapeutic action. Urea provides the composition with moisturizing and keratolytic properties. Further, it reduces itching and hyperkeratosis, which both common symptoms of psoriatic plaques. By including urea in the composition, it is believed that the side effects of the corticosteroid may be alleviated.

While urea may act as an antipruritic, it preferred to include an additional antipruritic in the composition. One common symptom of psoriasis is itching. As the skin may be sensitive to scratching, especially if being treated with a corticosteroid, cuts and subsequent infections may be counteracted by including an antipruritic in the composition. The antipruritic may be antihistamine, such as chlorpheniramine. Typically the antihistamine is a H₁-receptor antagonist. Except for chlorpheniramine there are various other examples of H₁-receptor antagonists, which may be included in the composition, such as diphenhydramine, thonzylamine, mepyramine, tenalidine, tripelenamine, chloropyramine, promethazine, tolpropamine, isothipendyl, and chlorphenoxamine.

Although, the skin damaging effect of the corticosteroid is being counteracted, the skin is anyhow sensitive and susceptible to infections. In order to counteract infections and to assist the skin in recovering, the composition comprises an antibiotic, such as fungicide or bactericide. While the tar extract provides the composition with antiseptic properties, it was anyhow found to be advantageous to include an antibiotic in the composition.

According to an embodiment the antibiotic is an anti-fungal agent, e.g. a fungicide. The fungicide is typically a fungicide being pharmaceutical acceptable for topical administration to the skin. A preferred example is tolnaftate. Except for tolnaftate there are various examples of fungicides which may be included in the composition, such as amorolfin, butenafine, naftifine, and terbinafine. Further the fungicide may be an imidazole, such as Bifonazole, Butoconazole, Clotrimazole, Econazole, Fenticonazole, Isoconazole, Ketoconazole, Miconazole, Omoconazole, Oxiconazole, Sertaconazole, Sulconazole, and Tioconazole, a triazole, such as albaconazole, fluconazole, Isavuconazole, Itraconazole, Posaconazole, Ravuconazole, Terconazole, and Voriconazole, or a thiazole such as Abafungin. The fungicide may also be a polyene macrolide fungicide, such as natamycin and nystatin.

Common medicaments of psoriasis include various ointments, creams, solutions and moisturizers. Typically a subject suffering from psoriasis typically has to apply various compositions in order to alleviate the disease. As several compositions are to be applied, the compliance may be low. As the disorder manifests it self by various symptoms over time and as the primary skin condition may give rise to secondary skin infections caused by bacteria and fungi, the disorder is complex to treat and manage. However, it is very difficult to formulate various components, having different physical properities (solubility, melting point, viscosity, etc.), into a single stable composition.

In order to provide a single formulation, the present composition is preferably formulated as an emulsion, as hydrophilic as well as hydrophobic constituents are to be present. Thus, the composition comprises an absorption base and water forming the basis for the emulsion. Further, it comprises propylene glycol. By including propylene glycol, constituents not readily soluble in water or oil may be included in the composition. Especially, propylene glycol assists in dispersing the main active ingredient, i.e. the corticosteroid, in the composition.

Further, the composition comprises lanolin. Lanolin acts emulsifying, emollient and moisturizing. More importantly, lanolin also stabilizes the emulsion, whereby coal tar may be added to the emulsion without disrupting its structure.

Absorption bases, such as oil/water absorption bases, are known in the art for making creams and ointments. An absorption base is an oleaginous base that contains an emulsifying agent. When water is taken up into the base, it will form an emulsion. Oil/water absorption bases typically can incorporate at least 45% of their volume of water. Water soluble ingredients may be added to the water phase of the emulsion, while non-water soluble ingredients may be added to the oil phase of the emulsion, provided that they are soluble in the oil phase. Insoluble constituents may be included mechanically into the emulsion.

According to a preferred embodiment, the absorption base is a self emulsifying oil/water absorption base. According to another embodiment, the absorption base is a water/oil absorption base. The emulsifying agent may be ionic as well as non-ionic. According to an embodiment, the emulsifying agent is non-ionic.

An example of a preferred absorption base is Neo-PCL O/W self emulsifying base. This absorption base comprises beeswax (Cera Alba), cetearyl, ethylhexanoate, stearyl slcohol, stearyl septanoate, cetyl palmitate, steareth-7, steareth-10, stearyl caprylate, myristyl alcohol.

Further the composition comprises water. By dispersing water in the absorption base an emulsion may be obtained. In order to prolong the shelf life of the composition it may further comprise a preservative, such as a paraben. Examples of suitable parabens are propyl 4-hydroxybenzoate and methyl 4-hydroxybenzoate.

According to an embodiment, the composition further comprises a vegetable oil, such as sweet almond oil. Sweet almond oil is a vegetable oil obtained by expression of the mature seeds of *Prunus dulcis.* It consists mainly of glycerides of oleic acid and smaller amounts of linoleic and palmitic acids. It has demulcent and emollient nutritional properties. Further and more importantly, a vegetable oil, such as sweet almond oil, was found to act synergistically with lanolin. By its own lanolin is not absorbed by the skin, but when mixed with a vegetable oil, such as sweet almond oil, it provides an emollient cream that penetrates the skin and facilitates the absorption of the active ingredients. Thereby, the composition softens the skin and assist in removing scale from psoriatic lesions more easily.

According to an embodiment, the composition further comprises ammonium lactate. Ammonium lactate is effective in preventing and treating dry skin. Further, it also helps to regenerate damaged skin. Without being bond to any theory, it is believed to contribute to restoring the skin and to counteract the skin damaging effects of the corticosteroid.

According to an embodiment, the composition further comprises glycerine. It may be used an adjuvant for the emulsion as it has lubricating, humectant and hygroscopic properties, which help the composition to stick to the skin and prevent water loss and subsequent drying of the emulsion over time. Further, it provides the composition with an emollient effect which in turn may improve the psoriatic lesion by softening the skin.

The various constituents may be present in the composition in various amounts. According to an embodiment, the composition comprises:
- 5 to 10 wt% of a tar extract;
- 0.01 to 2 wt% of a corticosteroid;
- 0.01 to 2 wt% of an antipruritic;
- 0.5 to 5 wt% of an antibiotic;
- 5 to 15 wt% urea;
- 10 to 30 wt% of an absorption base;
- 30 to 50 wt% water;
- 1 to 5 wt% lanolin;
- 1 to 10 wt% propylene glycol;
- optionally, 1 to 10 wt% vegetable oil;
- optionally 5 to 15 wt% of an aqueous solution of ammonium lactate comprising 70 wt% ammonium lactate; and
- optionally 1 to 10 wt% glycerin.

As already described the composition is effective in treating or soothing psoriasis lesions; especially lesions seen at subjects suffering from *psoriasis vulgaris.* According to an embodiment it is used for said purpose. Further, the composition may be used in a method of treating or soothing psoriasis lesions. According to another embodiment, the composition is used in the manufacture of a medicament for treating or soothing psoriasis lesion. When being used to treat or soothe psoriasis lesions, the composition is applied to areas of the skin affected by psoriasis. As the corticosteroid may damage the skin, it is preferred to avoid applying the composition to areas of the skin not affected by psoriasis. Typically, the composition is applied once a day in the evening prior to sleep. As the composition comprises a corticosteroid, the treatment is to be discontinued, once complete symptom relief has been accomplished. Symptom relief is typically seen within a week, implying the itching ceases, the scaling starts to decrease, etc. Complete symptom relief is typically seen within 1 to 3 months.

Subjects with severe psoriasis, previously not having been asymptomatic despite various types of treatments, including topical corticosteroids, typically unexpectedly experience complete symptom relief within 3 months. Further, they do usually remain asymptomatic for at least 6 months after withdrawal of the composition.

While the first composition comprises constituents counteracting the skin damaging effect of the corticosteroid, it may anyhow be advantageous to apply a second composition for minimizing the skin damaging effect of the corticosteroid. Typically, the first composition is very effective in removing the psoriasis lesions, whereby prolonged treatment not is necessary and very few side effects are thus seen during the treatment. However, in cases of severe psoriasis it may become necessary to prolong the treatment. Especially in such cases, it may be advantageous to apply a second composition for minimizing the skin damaging effects of the corticosteroid. According to an embodiment, such a second composition comprises hyaluronic acid, an absorption base and vegetable oil. The absorption base may of the same kind as the one used in the first composition.

An embodiment thus relates to kit comprising such a first composition for treating or soothing psoriasis lesions as already have been described herein, and a second composition comprising aqueous hyaluronic acid, an absorption base and vegetable oil. Typically, the compositions are applied separately; the first composition being administered once a day in the evening prior to sleep and the second one once a day in the morning.

The second composition has an emollient, lubricating and moisturizing effect on the skin. Furthermore and importantly, the second composition, due to the presence of hyaluronic acid, assists in regenerating and reconstructing collagen in the skin. Accordingly, also prolonged use of the first composition is possible without pronounced damages to the skin. It is to be observed that use of the second composition also may be advantageous in combination with first composition despite the treatment not being prolonged.

Furthermore, the second composition may also comprise vitamin E. The vegetable oil may comprise almond oil, argan oil, and/or macadamia oil. The second composition may also comprise a fragrance, such as peach essence.

As already described the first composition is effective in treating or soothing psoriasis lesions and the second one in minimizing the skin damaging effect of the corticosteroid. According to an embodiment the kit is used for treating or soothing psoriasis lesions. Further, the kit may be used in a method of treating or soothing psoriasis lesions. According to another embodiment, the kit is used in the manufacture of a medicament for treating or soothing psoriasis lesion. When being used to treat or soothe psoriasis lesions, the first composition is applied to areas of the skin affected by psoriasis. As the corticosteroid may damage the skin, it is preferred to avoid applying the first composition to areas of the skin not affected by psoriasis. Typically, the first composition is applied once a day in the evening prior to sleep, while the second composition is administered one once a day in the morning.

The second composition typically comprises 0.1 to 5 wt%, such as 0.5 to 2 wt%, hyaluronic acid. Further, it typically comprises 10 to 20 wt% of a vegetable oil or a mixture of vegetable oils.

Without further elaboration, it is believed that one skilled in the art can, using the preceding description, utilize the present invention to its fullest extent. The preferred specific embodiments described herein are, therefore, to be construed as merely illustrative and not limitative of the remainder of the description in any way whatsoever. Further, although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc do not preclude a plurality.

### Experimental

The following examples are mere examples and should by no mean be interpreted to limit the scope of the invention. Rather, the invention is limited only by the accompanying claims.

### Example 1 - Night cream

A composition comprising urea (100 g), saponified coal tar (70 ml), tolnaftate (20g), aqueous (70 %) ammonium lactate (100 ml), clobetasol dipropionate (5 g), chlorpheniramine maleate (6 g), propylene glycol (50 ml), lanolin (30 g), sweet almond oil (50 ml), neo-pcl o/w (175 g), glycerine (50 g), and aqua conservans (distilled water containing parabens: 0.1% sodium nipagin and 0.05% sodium nipasol) to 1000 g was prepared as indicated below.

In preparing the compositions the solid components were weighed and the liquids measured.

Firstly an aqueous phase and an oil phase were prepared.

Aqueous phase: The aqua conservans was measure in a test tube and poured into a 1-litre Erlenmeyer flask. Subsequently urea was added and the mixture was stirred until the urea had dissolved, whereupon chlorpheniramine maleate was added and the mixture was stirred until the chlorpheniramine maleate had dissolved. At last glycerine and an ammonium lactate solution was added to the aqueous solution.

Oil phase: Lanolin, Neo-PCL O/W and sweet almond oil was put into a wide-mouth 2-litre flask and mixed.

The flasks with the aqueous phase and the oil phase were both placed in a laboratory water bath at 60 to 70° C until both phases had reach the same temperature (about 65° C). At this temperature, both are in a liquid state.

Subsequently, the aqueous phase was gradually added to the oil phase under continuously stirring. Once both phases had been mixed, the resulting mixture was continuously stirred at constant speed until cooled to ambient temperature. The resulting mixture is an aqueous external phase emulsion (O/W emulsion).

Solid phase: Tolnaftate and clobetasol dipropionate were weighed into a mortar and mixed with a pestle to reduce their size into a fine dust. Then, propylene glycol was added and the resulting mixture mixed and grinded with pester until a homogeneous fluid mass was obtained.

Once a homogeneous fluid mass had been obtained, approximately 50 g of the aqueous external phase emulsion was added to the mortar and mixed with the homogeneous fluid mass. The resulting mixture was then added to the reaming part of the aqueous external phase emulsion. Finally, 100 ml saponified coal tar was measured into a test tube and mixed with the emulsion comprising the rest of the components using a mechanical stirrer to obtain the active night cream.

### Example 2 - Day Cream

A composition comprising 500 g of an aqueous solution of hyaluronic acid (2 vol%), comprising 10 vol% propylene glycol, vitamin E (α-tocopherol; 10 g), sweet almond oil (50 g), argan oil (50 g), macadamia oil (50 g), peach essence (a drop) and neo-pcl o/w to 1000 g was prepared as indicated below.

The aqueous solution of hyaluronic acid, comprising propylene glycol, was added to the Neo-PCL O/W and the resulting emulsion was stirred. Thereafter the oils were added. Then and α-tocopherol was added and at last peach essence. The day cream was obtained as a creamy emulsion.

### Example 3 - Treatment of subjects suffering from psoriasis

The night cream was applied areas of the skin affected by psoriasis once a day prior to sleep. Care was taken to only apply the potent cream to affected areas. In the morning the following day, the day cream was applied to the skin. As the day cream has an emollient, lubricating and moisturizing effect on the skin it may be used also at parts of the skin being unaffected by psoriasis.

### Subject I

A male subject, age 57, having psoriasis lesions (approx. size 10-15 cm) covering approximately 10% of his body, and suffering from severe itching, did apply the night cream and day cream above for 3 months.

The subject had been diagnosed with psoriasis for nearly 20 years ago and had previously used combinations of local corticosteroids and emollient and moisturizing creams. Further, the subject had also tried photo therapy. However, at the best those treatments alleviated the lesion somewhat. Further, the subject experienced side-effects (e.g. skin damage, such as skin thinning, changes in pigmentation, easy bruising, stretch marks, redness and dilated surface blood vessels) typically seen with treatment with corticosteroids.

Already after 1 week of administration of the night and day cream, the lesions were significantly reduced. Further, the itching has ceased. After 3 months of treatment the symptoms of psoriasis was gone. Despite withdrawal of the treatment, no symptoms of psoriasis were seen for nearly a year. The subject had previously not experienced complete symptom relief with conventional treatments.

### Subject 2

A male subject, age 59, having psoriasis lesions (approx. size 10-15 cm) covering approximately 2% of his body, and suffering from severe itching did apply the night cream and day cream above for 3 months.

The subject had been diagnosed with psoriasis for nearly 25 years ago and had previously used combinations of local corticosteroids and emollient and moisturizing creams. Further, the subject had also tried photo therapy as well as a topical tar based product (Skillingarydsalvan Original). However, at the best those treatments alleviated the lesion somewhat. Further, the subject experienced side-effects (e.g. skin damage, such as skin thinning, changes in pigmentation, easy bruising, stretch marks, redness and dilated surface blood vessels) typically seen with treatment with corticosteroids.

Already after 1 week of administration of the night and day cream, the lesions were significantly reduced. Further, the itching had ceased. After 3 months of treatment the symptoms of psoriasis was gone. Despite withdrawal of the treatment, no symptoms of psoriasis were seen for nearly a year

### Subject 3

A male subject, age 62, having psoriasis lesions (approx. size 10-15 cm) covering approximately 3 % of his body, and suffering from severe itching did apply the night cream and day cream above for 3 months.

The subject had been diagnosed with psoriasis for nearly 15 years ago and had previously used various types of treatments (e.g. emollient and moisturizing creams, photo therapy, topical tar based products etc.). However, at the best those treatments alleviated the lesions somewhat.

Already after 1 week of administration of the night and day cream, the lesions were significantly reduced. Further, the itching had ceased. After 3 months of treatment the symptoms of psoriasis was gone. Despite withdrawal of the treatment, no symptoms of psoriasis were seen for nearly a year.

### Evaluation

As can be seen from the examples provided above, the night cream, especially if combined with the day cream, was efficient in removing psoriasis lesions. It is to be noted that the subjects previously had used compositions comprising corticosteroids as well as compositions comprising tar extracts, in treating their psoriasis, but with limited effect. Further, the side effects typically seen with potent corticosteroid was significantly alleviated and the subjects did actually experience less severe side effect than the ones previously experienced with low-strength corticosteroid.

## Claims

1. A dermal composition comprising:
- a tar extract, such as saponified coal tar;
- a corticosteroid, such as clobetasol propionate;
- an antipruritic, such as an antihistamine, e.g. Chlorpheniramine;
- an antibiotic, such as an anti-fungal agent, e.g. tolnaftate;
- urea;
- an absorption base;
- water;
- lanolin; and
- propylene glycol.

2. The composition according to claim 1, wherein
- the corticosteroid is selected from the group consisting of: clobetasol propionate, betamethasone dipropionate, halobetasol propionate, fluocinonide, diflorasone diacetate, mometasone furoate, halcinonide, and desoximetasone;
- the antipruritic is an antihistamine selected from the group consisting of: chlorpheniramine, diphenhydramine, thonzylamine, mepyramine, tenalidine, tripelenamine, chloropyramine, promethazine, tolpropamine, isothipendyl, and chlorphenoxamine; and/or
- the antibiotic is an anti-fungal agent selected from the group consisting of: tolnaftate, amorolfin, butenafine, naftifine, and terbinafine.

3. The composition according to claim 1 or 2, wherein the absorption base is a self emulsifying oil/water absorption base.

4. The composition according to any one of the preceding claims, wherein the composition further comprises glycerin, a vegetable oil, such as sweet almond oil, ammonium lactate, and/or a preservative.

5. The composition according to any one of the preceding claims, wherein the composition comprises 5 to 10 wt% of the tar extract; 0.01 to 2 wt% of the corticosteroid; 0.01 to 2 wt% of the antipruritic; 0.5 to 5 wt% of the antibiotic; 5 to 15 wt% urea; 10 to 30 wt% of the absorption base; 30 to 50 wt% water; 1 to 5 wt% lanolin;
1 to 10 wt% propylene glycol; optionally, 1 to 10 wt% of a vegetable oil; optionally 5 to 15 wt% of an aqueous solution of ammonium lactate comprising 70 wt% ammonium lactate; and optionally 1 to 10 wt% glycerin.

6. The composition according to any one of the preceding claims, wherein the composition further comprises a preservative, wherein said preservative preferably is a paraben, such as propyl 4-hydroxybenzoate or methyl 4-hydroxybenzoate.

7. A composition according to any one of the preceding claims for use in treating or soothing psoriasis lesions by applying the composition to areas of the skin affected by psoriasis.

8. A composition according to any one of the preceding claims for use in treating or soothing psoriasis lesions by applying the composition to areas of the skin affected by psoriasis, wherein the composition is to be applied once a day in the evening prior to sleep.

9. A kit comprising two distinct compositions, wherein the first composition is a composition according to any one of the claims 1 to 6, and the second composition is a composition comprising aqueous hyaluronic acid, an absorption base, and vegetable oil.

10. The kit according to claim 9, wherein the second composition further comprises vitamin E, and optionally a fragrance, such as peach essence, and wherein said vegetable oil comprises almond oil, argan oil, and/or macadamia oil.

11. The kit according to claim 9 or 10, wherein:
the kit is for use in treating or soothing psoriasis lesions by separately applying the two compositions to a subject suffering from psoriasis;
the first composition is to be applied to areas of the skin affected by psoriasis; and
the first composition is to be administered once a day in the evening prior to sleep and the second one once a day in the morning.

## Patentansprüche

1. Dermale Zusammensetzung, umfassend:
- einen Teerextrakt wie verseiften Kohlenteer;
- sein Kortikosteroid wie Clobetasol-Proprionat;
- ein Antijuckreizmittel wie ein Antihistamin, z.B. Chlorpheniramin;
- ein Antibiotikum wie ein Antipilzmittel, z.B. Tolnaftat;
- Harnstoff;
- eine Absorptionsbasis;
- Wasser;
- Lanolin; und
- Propylenglykol.

2. Zusammensetzung gemäß Anspruch 1, wobei
- das Kortikosteroid ausgewählt ist aus der Gruppe, bestehend aus: Clobetasolproprionat, Betamethasondipropionat, alobetasolpropionat, Fluocinonid, Diflorasondiacetat, Mometasonfuroat, Halcinonid und Desoximetason;
- dans Antijuckreizmittel ein Antihistamin ist, ausgewählt aus der Gruppe, bestehend aus: Chlorpheniramin, Diphenhydramin, Thonzylamin, Mepyramin, Tenalidin, Tripelenamin, Chloropyramin, Promethazin, Tolpropamin, Isothipendyl und Chlorphenoxamin; und/oder
- das Antibiotikum ein Antipilzmittel ist, ausgewählt aus der Gruppe, bestehend aus: Tolnaftat, Amorolfin, Butenafin, Naftifin und Terbinafin.

3. Zusammensetzung gemäß Anspruch 1 oder 2, wobei die Absorptionsbasis eine selbstemulgierende Öl/Wasser-Absorptionsbasis ist.

4. Zusammensetzung gemäß einem der vorhergehenden Ansprüche, wobei die Zusammensetzung weiterhin Glycerin, ein pflanzliches Öl wie Süßmandelöl, Ammoniumlactat und/oder ein Konservierungsmittel umfasst.

5. Zusammensetzung gemäß einem der vorhergehenden Ansprüche, wobei die Zusammensetzung 5 bis 10 Gew.% des Teerextraktes; 0,01 bis 2 Gew.% des Kortikosteroids; 0,01 bis 2 Gew.% des Antijuckreizmittels; 0,5 bis 5 Gew.% des Antibiotikums; 5 bis 15 Gew.% Harnstoff; 10 bis 30 Gew.% der Absorptionsbasis; 30 bis 50 Gew.% Wasser; 1 bis 5 Gew.% Lanolin; 1 bis 10 Gew.% Propylenglycol; gegebenenfalls 1 bis 10 Gew.% eines pflanzliche Öles; gegebenenfalls 5 bis 15 Gew.% einer wässrigen Lösung von Ammoniumlactat, umfassend 70 Gew.% Ammoniumlactat; gegebenenfalls 1 bis 10 Gew.% Glycerin umfasst.

6. Zusammensetzung gemäß einem der vorhergehenden Ansprüche, wobei die Zusammensetzung weiterhin ein Konservierungsmittel umfasst, wobei das Konservierungsmittel vorzugsweise Paraben wie Propyl-4-hydroxybenzoat oder Methyl-4-hydroxybenzoat ist.

7. Zusammensetzung gemäß einem der vorhergehenden Ansprüche zur Verwendung bei der Behandlung oder Beruhigung von Psoriasis-Läsionen durch Anwenden der Zusammensetzung auf Bereiche der Haut, die von Psoriasis betroffen sind.

8. Zusammensetzung gemäß einem der vorhergehenden Ansprüche zur Verwendung bei der Behandlung oder Beruhigung von Psoriasis-Läsionen durch Anwenden der Zusammensetzung auf Bereiche der Haut, die von Psoriasis betroffen sind, wobei die Zusammensetzung einmal pro Tag am Abend vor dem Schlafen anzuwenden ist.

9. Kit, umfassend zwei getrennte Zusammensetzungen, wobei die erste Zusammensetzung eine Zusammensetzung gemäß einem der Ansprüche 1 bis 6 ist und die zweite Zusammensetzung eine Zusammensetzung ist, die wässrige Hyaluronsäure, eine Absorptionsbasis und pflanzliches Öl umfasst.

10. Kit gemäß Anspruch 9, wobei die zweite Zusammensetzung weiterhin Vitamin E und gegebenenfalls einen Duftstoff wie Pfirsichessenz umfasst und wobei das pflanzliche Öl Mandelöl, Arganöl und/oder Macadamiaöl umfasst.

11. Kit gemäß Anspruch 9 oder 10, wobei:
der Kit für die Verwendung bei der Behandlung oder Beruhigung von Psoriasis-Läsionen durch getrennte Anwendung der zwei Zusammensetzungen bei einem Individuum ist, das an Psoriasis leidet;
die erste Zusammensetzung bei Bereichen der Haut, die von Psoriasis betroffen sind, anzuwenden ist; und
die erste Zusammensetzung einmal am Tag am Abend vor dem Schlafen anzuwenden ist und die zweite einmal am Tag am Morgen anzuwenden ist.

## Revendications

1. Composition dermique comprenant :
- un extrait de goudron, tel qu'un goudron de houille saponifié ;
- un corticostéroïde, tel que du propionate de clobétasol ;
- un antiprurigineux, tel qu'un antihistaminique, par exemple de la Chlorphéniramine ;
- un antibiotique, tel qu'un agent antifongique, par exemple du tolnaftate ;
- de l'urée ;
- une base absorbante ;
- de l'eau ;
- de la lanoline ; et
- du propylène glycol.

2. Composition selon la revendication 1, dans laquelle
- le corticostéroïde est choisi dans le groupe constitué de : propionate de clobétasol, dipropionate de bêtaméthasone, propionate d'halobétasol, fluocinonide, diacétate de diflorasone, furoate de mométasone, halcinonide et désoximétasone ;
- l'antiprurigineux est un antihistaminique choisi dans le groupe constitué de : chlorphéniramine, diphenhydramine, thonzylamine, mépyramine, tenalidine, tripélénamine, chloropyramine, prométhazine, tolpropamine, isothipendyl et chlorphénoxamine ; et/ou
- l'antibiotique est un agent antifongique choisi dans le groupe constitué de : tolnaftate, amorolfine, buténafine, naftifine et terbinafine.

3. Composition selon la revendication 1 ou 2, dans laquelle la base absorbante est une base absorbant l'huile/l'eau auto-émulsifiante.

4. Composition selon l'une quelconque des revendications précédentes, dans laquelle la composition comprend en outre de la glycérine, une huile végétale, telle que de l'huile d'amande douce, du lactate d'ammonium et/ou un agent de conservation.

5. Composition selon l'une quelconque des revendications précédentes, dans laquelle la composition comprend de 5 à 10 % en poids de l'extrait de goudron ; de 0,01 à 2 % en poids du corticostéroïde ; de 0,01 à 2 % en poids de l'antiprurigineux ; de 0,5 à 5 % en poids de l'antibiotique ; de 5 à 15 % en poids d'urée ; de 10 à 30 % en poids de la base absorbante ; de 30 à 50 % en poids d'eau ; de 1 à 5 % en poids de lanoline ; de 1 à 10 % en poids de propylène glycol ; éventuellement, de 1 à 10 % en poids d'une huile végétale ; éventuellement de 5 à 15 % en poids d'une solution aqueuse de lactate d'ammonium comprenant 70 % en poids de lactate d'ammonium ; et éventuellement de 1 à 10 % en poids de glycérine.

6. Composition selon l'une quelconque des revendications précédentes, dans laquelle la composition comprend en outre un agent de conservation, dans laquelle ledit agent de conservation est de préférence un parabène, tel que du 4-hydroxybenzoate de propyle ou du 4-hydroxybenzoate de méthyle.

7. Composition selon l'une quelconque des revendications précédentes pour une utilisation dans le traitement ou le soulagement des lésions du psoriasis par application de la composition sur les zones de la peau affectées par le psoriasis.

8. Composition selon l'une quelconque des revendications précédentes pour une utilisation dans le traitement ou le soulagement des lésions du psoriasis par application de la composition sur les zones de la peau affectées par le psoriasis, dans laquelle la composition doit être appliquée une fois par jour le soir avant le coucher.

9. Kit comprenant deux compositions distinctes, dans lequel la première composition est une composition selon l'une quelconque des revendications 1 à 6, et la deuxième composition est une composition comprenant un acide hyaluronique aqueux, une base absorbante et une huile végétale.

10. Kit selon la revendication 9, dans lequel la deuxième composition comprend en outre de la vitamine E, et éventuellement un parfum, tel qu'une essence de pêche, et dans lequel ladite huile végétale comprend une huile d'amande, une huile d'argan et/ou une huile de macadamia.

11. Kit selon la revendication 9 ou 10, dans lequel :
le kit est destiné à une utilisation dans le traitement ou le soulagement des lésions du psoriasis par application séparée des deux compositions sur un sujet souffrant du psoriasis ;
la première composition doit être appliquée sur les zones de la peau affectées par le psoriasis ; et
la première composition doit être administrée une fois par jour le soir avant le coucher et la deuxième une fois par jour le matin.
